# EUROPEAN PATENT SPECIFICATION

(11) **EP 0 646 566 B1**
(45) Date of publication and mention of the grant of the patent: **19.11.1997**
(21) Application number: 94307194.4
(22) Date of filing: 30.09.1994
(51) Int. Cl.: C07C 51/58, C07D 213/78

(54) **Process for the preparation of acid chloride compounds**
Verfahren zur Herstellung von Säurechloriden
Procédé pour la préparation de chlorides d'acides

(30) Priority: 05.10.1993 EP 93307903
(43) Date of publication of application: 05.04.1995
(73) Proprietor: SHELL INTERNATIONALE RESEARCH MAATSCHAPPIJ B.V., 2596 HR Den Haag (NL)
(72) Inventor: Bull, Michael John, Sittingbourne, Kent ME10 4TY (GB); Cornforth, John Warcup, Lewes, Sussex (GB)
(74) Representative: Allam, Peter Clerk

(56) References cited:
- EP-A- 0 447 004
- US-A- 1 557 154
- CHEMICAL ABSTRACTS, vol. 91, no. 5, 30 July 1979, Columbus, Ohio, US; abstract no. 39152e, K. KAMATA 'Benzoyl chloride' page 603 ;
- JOURNAL OF ORGANIC CHEMISTRY., vol.25, July 1960, EASTON US pages 1115 - 1118 M.E. HILL 'Metal halide catalyzed hydrolysis of trichloromethyl compounds'

## Description

This invention relates to a process for the preparation of acid chloride compounds.

J.C.S. Chem.Comm (1977), 808-9 discloses a process for preparing aromatic acid chlorides. The process involves reacting substituted benzylidyne chlorides with hexamethyldisiloxane in the presence of iron (III) chloride to produce the corresponding substituted benzoyl chloride. In the reaction, trimethylchlorosilane is produced which must be hydrolysed back to hexamethyldisiloxane for re-use.

J.O.C. (1960), 1115-7 discloses a process of metal halide catalysed hydrolysis of trichloromethyl compounds to form acids. For example, the document discloses that (trichloromethyl)benzene may be reacted with water in the presence of ferric chloride and chloroform as a solvent to produce benzoic acid.

US 1557154 discloses a process for the manufacture of benzoyl chloride in which trichloromethylbenzene is hydrolysed in the presence of a catalyst comprising an anhydrous zinc salt such as anhydrous zinc chloride.

This invention is based upon the discovery of a novel process for preparing acid chloride compounds.

According to a first aspect of the present invention, there is provided a process for preparing a compound of general formula 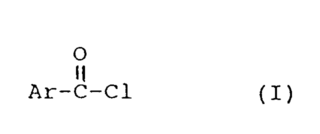 where Ar represents an optionally substituted aromatic or heteroaromatic group, the process comprising reacting a compound of general formula

Ar-C(L¹)₂Cl (II)

where each L¹ independently represents a leaving group, with water in the presence of a Lewis acid and a solvent characterised in that the solvent is 1,2-dichloroethane.

It is believed that the two chlorine atoms of the solvent may form a transition state or intermediate in the reaction which aids the preferential formation of the desired acid chloride of general formual I.

Preferably, each L¹ independently represents a halogen atom. Preferred halogen atoms are chlorine, bromine and iodine atoms. Preferably, both of said groups L¹ represent a chlorine atom.

Said Lewis acid is preferably a weak Lewis acid. Said Lewis acid is preferably a halide of a transition metal. Preferably, said Lewis acid is a chloride of a transition metal. Preferred transition metals include iron, gallium and antimony. Preferred Lewis acids include FeCl₃, GaCl₃ and SbCl₅. Most preferably, the Lewis acid is ferric chloride (FeCl₃).

Said Lewis acid, for example ferric chloride, is preferably substantially anhydrous.

Preferably, a catalytic amount of said Lewis acid is added in said process. For example, the molar ratio of said Lewis acid to said compound of general formula II is preferably less than 0.15 and, more preferably, is less than 0.1.

Preferably, water is of low solubility in the solvent. 1,2-Dichloroethane has a solubility of 0.15g/100g water at 25°C (Techniques of Organic Chemistry, vol. VII, Organic Solvents) and has therefore been found to be a surprisingly advantageous solvent for use in the preferential formation of the desired acid chloride of general formula I.

Preferably, in the process of the first aspect, approximately equimolar amounts of the compound of general formula II and water are reacted together. Preferably, in the process, the compound of general formula II and the Lewis acid are mixed together in said solvent, suitably at an elevated temperature, preferably under reflux, Said water is preferably added to the mixture over an extended period of time. During the addition, the reaction mixture is preferably refluxed. Said water is preferably added to the reaction mixture so that all added water dissolves in the reaction mixtures. Dean and Stark apparatus is of utility in achieving this aim. With Dean and Stark apparatus, water is dissolved in the solvent of the reaction mixture so that the solvent is saturated with water.

After the added water has reacted with the compound of general formula II, the Lewis acid may be removed from the mixture, for example, by filtration.

The compound of general formula I may be isolated by standard techniques. Alternatively, the compound of general formula I in the reaction mixture may be further reacted. For example, the process of the first aspect may be of utility in the preparation of herbicidal carboxamide derivatives described in European Patent Application No. 0 447 004 (Shell), In this case, preferably the group Ar in said compound of general formula I represents an optionally substituted heteroaromatic group. Preferably, said optionally substituted heteroaromatic group is an optionally substituted pyridyl group of general formula 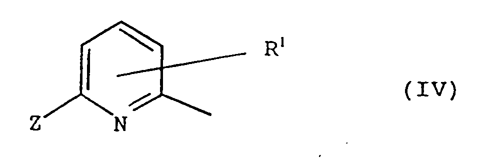 where R¹ represents a hydrogen or halogen atom or an alkyl or haloalkyl group and Z represents a halogen atom. Preferably, in said pyridyl group of general formula IV, Z represents a chlorine atom and R¹ represents a hydrogen atom.

Preferably, the further reaction of said compound of general formula I involves reacting the compound of general formula I with a compound of general formula 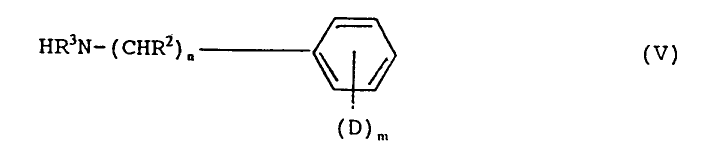 where R² represents a hydrogen atom or an alkyl group; R³ represents a hydrogen atom or an alkyl or alkenyl group; the or each group D independently represents a halogen atom or an alkyl, nitro, cyano, haloalkyl, alkoxy or haloalkoxy group; n represents 0 or 1; and m represents 0 or an integer from 1 to 5, to prepare a compound of general formula

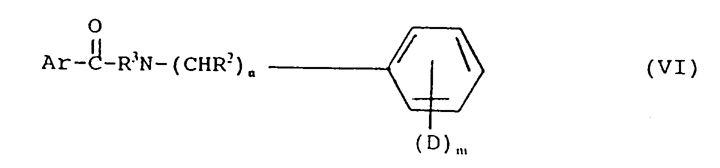

Preferably, in said compound of general formula VI, R³ represents a hydrogen atom, m represents 1, D represents a fluorine atom in the 4- position relative to the amine group, and n represents 0.

Said compound of general formula VI may be further reacted to prepare compounds of general formula 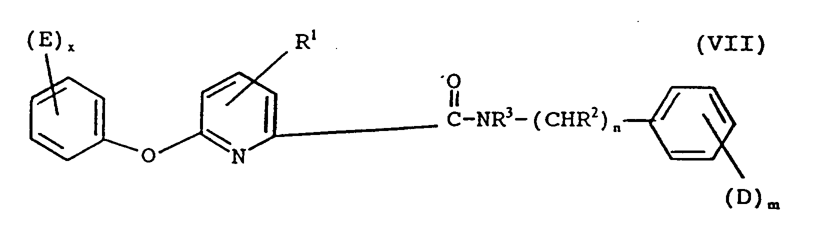 where R¹, R², R³, D, n and m are as described in any statement herein, each group E independently represents a halogen atom or an optionally substituted alkyl, alkoxy, alkenyloxy, alkynylory, cyano, carboxy, alkoxycarbonyl, alkylthiocarbonyl, alkylcarbonyl, amide, alkylamido, nitro, alkylthio, haloalkylthio, alkenylthio, alkynylthio, alkylsulphinyl, alkylsulphonyl, alkyloximinoalkyl or alkenyloximinoalkyl group, and x represents 0 or an integer from 1 to 5.

According to a second aspect of the invention, there is provided a process for preparing a compound of general formula VI, the process including the process step of said first aspect.

The invention will now be further described, with reference to the following Examples.

Examples 1 and 4 describe the preparation of certain acid chloride compounds. Examples 2, 3 and 5 describe subsequent reactions of the acid chloride compounds.

### EXAMPLE 1

### Preparation of 2-chloro-6-pyridinecarbonyl chloride

[Ar = 2-chloro-6-pyridyl in the compound of general formula I]

Nitrapyrin [2-chloro-6-(trichloromethyl)pyridine; Ar = 2-chloro-6-pyridyl and L¹ = L² = Cl in the compound of general formula II] (46.2g; 0.2M), 1,2-dichloroethane (1 litre) and anhydrous ferric chloride (FeCl₃) (8.1g; 0.04M) were stirred under reflux for half-an-hour using a Dean and Stark separator. Water (3.6g) was then added to the Dean and Stark separator and the reaction mixture was stirred under reflux for 24 hours, by which time all the water had been consumed. Gas-liquid chromatography showed 93% product on the treatment with 4-fluoroaniline (see Example 2). The ferric chloride was then filtered off and the filtrate concentrated.

### EXAMPLE 2

### Preparation of N-(4-fluorophenyl)-2-chloro-6-pyridinecarboxamide

To the concentrated filtrate of Example 1 was added 4-fluoroaniline (28g; 0.25M) at 20-70°C. The mixture was then stirred under reflux for ¾ hr, by which time gas evolution had ceased. The mixture was then cooled to 20°C, washed with dilute hydrochloric acid and stripped to give a reddish brown oil (50.5g) which was then dissolved in dichloromethane and passed through a SiO₂ pad to give the desired product (37.3g; yield 74% based on Nitrapyrin added). Gas-liquid chromatography showed 99% pure.

### EXAMPLE 3

### Preparation of N-(fluorophenyl)-2-(3-α,α,α-trifluoromethyl phenoxy)-6-pyridinecarboxamide

To a slurry of potassium carbonate (435g; 3.15 moles) in dimethylformamide (1.8 litres) was added the pyridinecarboxamide of Example 2 (752g; 3.0 moles) and 3-trifluoromethylphenol (502g; 3.1 moles) and the mixture brought to reflux under nitrogen for 5 hours. Evolution of carbon dioxide began at ca. 120°C. After cooling, the reaction mixture was added to 0.6M hydrochloric acid (10.5 litres) and extracted with methylene chloride (2 x 2.5 litres). The organic extracts were combined and backwashed with water (10 litres) and the solvent flashed. The residue, after decolouration through a short column of silica gel, was recrystallised from cyclohexane/isopropanol (1:3; 4.7 litres) to give the title compound (835g; 74% yield) mp. 105-107°C. cyclohexane/isopropanol (1:3; 4.7 litres) to give the title compound (835g; 74% yield) mp. 105-107°C.

### EXAMPLE 4

### Preparation of benzoyl chloride

α,α,α-Trichlorotoluene (19.6g; 0.1 M), ferric chloride (4.1g; 0.02M) and 1,2-dichloroethane (500ml) were stirred under reflux under heavier than water Dean and Stark apparatus. Water (1.8g) was then added to the Dean and Stark apparatus and the mixture was refluxed for 4 hours. By this time, all of the water had been consumed. The mixture was then filtered through Hyflo (Trade Mark) to remove the ferric chloride.

### EXAMPLE 5

### Preparation of N-(4-fluorophenyl)phenylcarboxamide

To the filtrate of Example 4 was added 4-fluoroaniline (22.2g; 0.22M) with stirring over 15 minutes. Dilute hydrochloric acid was then added, the organic layer separated and the remainder was stripped to give a dark red solid (25 g). The solid was dissolved in dichloromethane and passed through a SiO₂ pad to give the title compound (6.3g; 29% yield).

## Claims

1. A process for preparing a compound of general formula 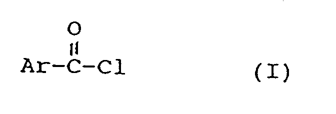 where Ar represents an optionally substituted aromatic or heteroaromatic group, the process comprising reacting a compound of general formula
Ar-C(L¹)₂Cl (II)
where each L¹ independently represents a leaving group, with water in the presence of a Lewis acid and a solvent characterised in that the solvent is 1,2-dichloroethane.

2. A process according to Claim 1, where each L¹ independently represents a halogen atom.

3. A process according to Claim 1 or Claim 2, where both of said groups L¹ represent a chlorine atom.

4. A process according to any preceding claim, where the Lewis acid is ferric chloride (FeCl₃).

5. A process according to any preceding claim, where said group Ar is an optionally substituted pyridyl group of general formula 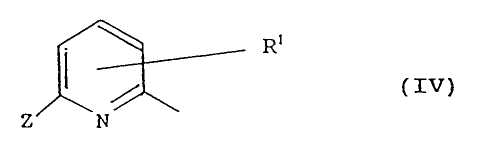 where R¹ represents a hydrogen or halogen atom or an alkyl or haloalkyl group and Z represents a halogen atom.

6. A process for preparing a compound of general formula 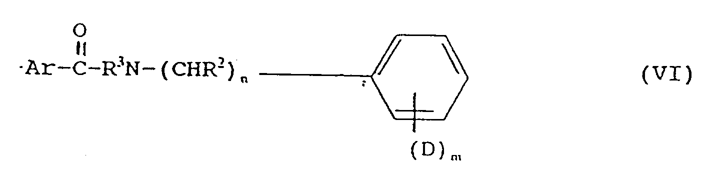 where R² represents a hydrogen atom or an alkyl group; R³ represents a hydrogen atom or an alkyl or alkenyl group; the or each group D independently represents a halogen atom or an alkyl, nitro, cyano, haloalkyl, alkoxy or haloalkoxy group; n represents 0 or 1; and m represents 0 or an integer from 1 to 5, the process including, as a process step, a process according to any of Claims 1 to 5.

## Patentansprüche

1. Verfahren zur Herstellung einer Verbindung der allgemeinen Formel 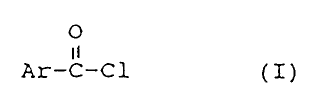 in der Ar eine gegebenenfalls substituierte aromatische oder heteroaromatische Gruppe darstellt, wobei das Verfahren umfaßt, daß man eine Verbindung der allgemeinen Formel
Ar-C(L¹)₂Cl (II)
in der jedes L¹ unabhängig eine Abgangsgruppe darstellt, mit Wasser in Anwesenheit einer Lewis-Säure und eines Lösungsmittels umsetzt, dadurch gekennzeichnet, daß das Lösungsmittel 1,2-Dichlorethan ist.

2. Verfahren nach Anspruch 1, in dem jedes L¹ unabhängig ein Halogenatom darstellt.

3. Verfahren nach Anspruch 1 oder Anspruch 2, in dem beide Gruppen L¹ ein Chloratom darstellen.

4. Verfahren nach irgendeinem vorangehenden Anspruch, in dem die Lewis-Säure Eisen(III)chlorid (FeCl₃) ist.

5. Verfahren nach irgendeinem vorangehenden Anspruch, in dem die Gruppe Ar eine gegebenenfalls substitiuierte Pyridylgruppe der allgemeinen Formel 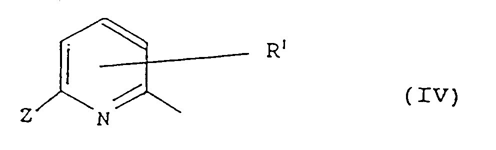 ist, in der R¹ ein Wasserstoff- oder Halogenatom oder eine Alkyl- oder Halogenalkylgruppe darstellt und Z ein Halogenatom darstellt.

6. Verfahren zur Herstellung einer Verbindung der allgemeinen Formel 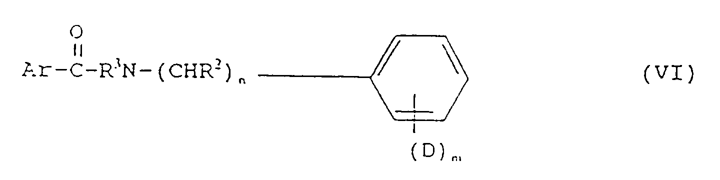 in der R² ein Wasserstoffatom oder eine Alkylgruppe darstellt; R³ ein Wasserstoffatom oder eine Alkyl- oder Alkenylgruppe darstellt; die oder jede Gruppe D unabhängig ein Halogenatom oder eine Alkyl-, Nitro-, Cyano-, Halogenalkyl-, Alkoxy- oder Halogenalkoxygruppe darstellt; n 0 oder 1 darstellt; und m 0 oder eine ganze Zahl von 1 bis 5 darstellt, wobei das Verfahren als Verfahrensschritt ein Verfahren nach irgendeinem der Ansprüche 1 bis 5 einschließt.

## Revendications

1. Un procédé de préparation d'un composé répondant à la formule générale 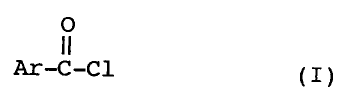 où Ar représente un groupe aromatique ou hétéroaromatique, éventuellement substitué, ledit procédé comprenant le fait de faire réagir un composé répondant à la formule générale
Ar-C(L¹)₂Cl (II)
où chaque L¹ représente indépendamment un groupe labile, avec de l'eau en présence d'un acide de Lewis et d'un solvant caractérisé en ce que le solvant est le 1,2-dichloroéthane.

2. Un procédé selon la revendication 1, où chaque L¹ représente indépendamment un atome d'halogène.

3. Un procédé selon la revendication 1 ou la revendication 2, où l'un et l'autre desdits groupes L¹ représentent un atome de chlore.

4. Un procédé selon l'une quelconque des revendications précédentes, où l'acide de Lewis est le chlorure ferrique (FeCl₃).

5. Un procédé selon l'une quelconque des revendications précédentes, où ledit groupe Ar est un groupe pyridyle éventuellement substitué répondant à la formule générale 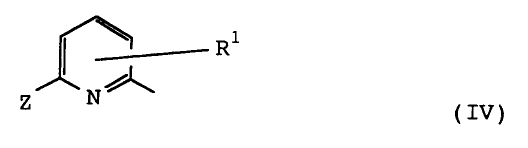 où R¹ représente un atome d'hydrogène ou d'halogène ou un groupe alkyle ou halogénoalkyle, et Z représente un atome d'halogène.

6. Un procédé de préparation d'un composé de formule générale 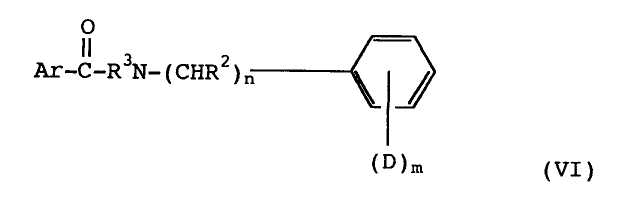 où R² représente un atome d'hydrogène ou un groupe alkyle; R³ représente un atome d'hydrogène ou un groupe alkyle ou alcényle; le ou chaque groupe D représente indépendamment un atome d'halogène ou un groupe alkyle, un groupe nitro, un groupe cyano, un groupe halogénoalkyle, un groupe alcoxy ou un groupe halogénoalcoxy; n vaut 0 ou 1; et m vaut 0 ou est un nombre entier compris entre 1 et 5, ledit procédé comprenant, en tant qu'étape de procédé, un procédé selon l'une quelconque des revendications 1 à 5.
